# EUROPEAN PATENT SPECIFICATION

(11) **EP 2 282 785 B1**
(45) Date of publication and mention of the grant of the patent: **31.12.2014**
(21) Application number: 09738960.5
(22) Date of filing: 28.04.2009
(51) Int. Cl.: A61L 27/38, A61F 2/02, A61L 27/60, A61F 2/10

(54) **HEPARIN-CONJUGATED FIBRIN GEL AND METHOD AND KIT FOR PREPARING THE SAME**
HEPARIN-KONJUGIERTES FIBRINGEL SOWIE VERFAHREN UND KIT ZU SEINER HERSTELLUNG
GEL DE FIBRINE CONJUGUÉ À L HÉPARINE ET PROCÉDÉ ET KIT POUR PRÉPARER CELUI-CI

(30) Priority: 28.04.2008 KR 20080039322; 24.04.2009 KR 20090035779
(43) Date of publication of application: 16.02.2011
(73) Proprietor: Samyang Corporation, Seoul (KR)
(72) Inventor: KIM, Byung-Soo, Seoul 135-280 (KR); JEON, O-Ju, Seoul 133-020 (KR); YANG, Hee-Seok, Seoul 138-200 (KR)
(74) Representative: Müller & Schubert
(86) International application number: PCT/KR2009/002220
(87) International publication number: WO 2009/134054

(56) References cited:
- EP-A2- 0 325 270
- WO-A1-01/66164
- WO-A1-02/085422
- US-A- 4 925 677
- US-A1- 2006 128 948
- US-A1- 2006 172 008

## Description

### Technical Field

The present invention relates to tissue engineering focusing on repair of damaged tissue and organs, and more particularly, to regeneration of tissues associated with transferring growth factors.

Specifically, a method of preparing a fibrin gel conjugated with injectable heparin containing a growth factor is provided, in which activated heparin conjugated with fibrinogen is mixed with free fibrinogen and dissolved, which is then mixed with thrombin, thereby completing an injectable heparin-conjugated fibrin gel.

Meanwhile, a growth factor having an affinity for heparin physically binds to the heparin-conjugated fibrin gel prepared according to the above-mentioned method of the present invention and is injected into a human body, thereby resulting in providing sustained release of growth factors for a long period of time. Therefore, it can be used as a therapeutic drug for stimulating regeneration of tissue, such as bones, skin, blood vessels, cartilages, etc.

### Background Art

Tissue engineering is a research field focusing on repair of tissues and organs damaged due to diseases or accidents. Methods of using an injectable gel as a matrix for regenerating tissues are being widely researched, and may reduce recovery time, pain and costs imposed on patients since a drug can be easily injected into a target site with no surgical procedures. Examples of the injectable gels used so far include collagen gels, fibrin gels and alginic acid gels. Particularly, since the fibrin gel can be obtained from a patient s blood, it does not bring about an immune reaction. Therefore, it is receiving great attention as an autologous matrix for tissue engineering.

Fibrin is a kind of scleroprotein, which is non-soluble protein generated by reaction of fibrinogen in a plasma and an enzyme, thrombin. That is, fibrin is prepared in a gel type by reaction with fibrinogen in the presence of thrombin at room temperature through enzymatic polymerization. The fibrin gel prepared as such is significant to tissue engineering focusing on repair of damaged tissues and organs. Particularly, the fibrin gel facilitates delivery of growth factors, and therefore various researches into fibrin gels are being conducted to regenerate tissue such as bones, skin, blood vessels, cartilages, etc.

Since heparin, a kind of glycosaminoglycan, contains a large quantity of sulfate groups, thereby being highly negative-charged, it is often used as an affinity ligand conjugated with specific heparin-conjugated protein. The characteristic of the affinity ligand is also applied to chromatography for purifying protein. However, recent attention has focused on applying this characteristic to fix heparin-conjugated protein to a carrier of a protein drug to specifically deliver to a specific site in a body. WO0166164 discloses heparin non-covalently conjugated to fibrin trough heparin binding peptides incorporated in the fibrin matrix.

### Disclosure of Invention

### Technical Solution

The present invention is directed to a simple method of preparing a fibrin gel directly conjugated with heparin, as defined in claim 1, thereby having long-term, sustained release of a contained drug.

The present invention is also directed to a protein carrier including a fibrin gel directly conjugated with heparin, thereby having long-term, sustained release of a contained drug, and a kit for preparing a fibrin gel as described above.

To this end, the present inventors have conducted research into a fibrin gel composition capable of containing a large quantity of growth factors without using a peptide, thereby resulting in preparation of fibrinogen directly conjugated with heparin, which is activated before conjugation.

In addition, it was found that a fibrin gel could not easily be obtained by crosslinking polymerization of heparin-directly conjugated fibrinogens only, but a fibrin gel allowing excellent sustained release of a contained drug and easy handling could be obtained by crosslinking polymerization of both fibrinogens directly conjugated with heparins and free fibrinogens, which are not conjugated with heparins.

Accordingly, in one aspect of the present invention, a method of preparing a heparin-conjugated fibrin gel is provided, which includes: activating heparin; conjugating the activated heparin with fibrinogen to prepare heparin-conjugated fibrinogen; mixing the heparin-conjugated fibrinogen prepared in the previous step with free fibrinogen to prepare a fibrinogen mixture; and mixing the fibrinogen mixture prepared in the previous step with thrombin.

A kit for preparing a heparin-conjugated fibrin gel, including heparin-conjugated fibrinogen, free fibrinogen which is not conjugated with heparin, and thrombin, and a growth factor carrier including a fibrin gel containing fibrinogen directly conjugated with heparin and a growth factor are provided.

According to the method of preparing the heparin-conjugated fibrin gel, the heparin-conjugated fibrin gel having an affinity for drugs such as growth factors may be easily prepared at low costs, and can also be used as a therapeutic drug excellently effective on generation of tissues such as bones, skin, blood vessels, cartilages, etc. by sustainably releasing drugs such as growth factors to a local site for a long period of time through injection into a human body.

### Brief Description of Drawings

These and/or other objects, aspects and advantages of the invention will become apparent and more readily appreciated from the following description of the exemplary embodiments, taken in conjunction with the accompanying drawings of which:
FIG. 1 shows NMR data obtained to confirm whether activated heparin is conjugated with fibrinogen;
FIG. 2 shows FTIR data obtained to confirm whether activated heparin is conjugated with fibrinogen;
FIG. 3 is a graph showing a release behavior of bone morphogenetic protein-2 (BMP-2) in an injectable heparin-conjugated fibrin gel composition;
FIG. 4 is a photograph showing formation of gels using mixtures of heparin-free fibrinogen or heparin-conjugated fibrinogen with thrombin solutions through crosslinking polymerization, respectively;
FIG. 5 is photographs showing formation of gels according to increasing contents of heparin-free fibrinogen; and
FIG. 6 is a graph showing a release behavior of platelet-derived growth factor (PDGF) in an injectable heparin-conjugated PRP fibrin gel composition.

### Best Mode for Carrying out the Invention

Hereinafter, the present invention will be described in more detail with reference to exemplary embodiments.

The present invention provides a method of preparing a heparin-conjugated fibrin gel, which includes: activating heparin; conjugating the activated heparin with fibrinogen to prepare heparin-conjugated fibrinogen; mixing the heparin-conjugated fibrinogen prepared in the previous step with free fibrinogen to prepare a fibrinogen mixture; and mixing the fibrinogen mixture prepared in the previous step with thrombin. The fibrin gel prepared according to the present invention may be an injectable fibrin gel.

According to the method of preparing a heparin-conjugated fibrin gel of the present invention, the fibrinogen may be derived from a mammal, and preferably, human plasma.

The term 'heparin-conjugated fibrinogen' denotes fibrinogen conjugated with heparin prepared by activation of heparin described herein, and the term 'free fibrinogen' or 'fibrinogen' individually used herein denotes fibrinogen which is not conjugated with other compounds, particularly, heparin.

The heparin used in the present method may have a low molecular weight, which may be in the range of 1000 to 20000.

Activation of the heparin in the method of preparing a fibrin gel according to the present invention is to introduce a NHS- group to the heparin, which may be conducted by any method. While the activation of the heparin may be performed by reaction of the heparin, and carbodiimide and N-hydroxysuccinimide, and activated into NHS-heparin, the present invention is not limited thereto.

Preparation of the heparin-conjugated fibrinogen in the method of preparing a fibrin gel according to the present invention is accomplished by reaction between the NHS-heparin and fibrinogen. Specifically, the heparin-conjugated fibrinogen is prepared by reaction between a carboxy group of the NHS-heparin and an amino group present in protein of the fibrinogen. Meanwhile, the heparin-conjugated fibrinogen prepared by the above-mentioned reaction may be readily used for the subsequent step, or stored in a liquid or dry powder type for future use depending on its purpose. Preferably, the heparin-conjugated fibrinogen may be lyophilized and easily stored.

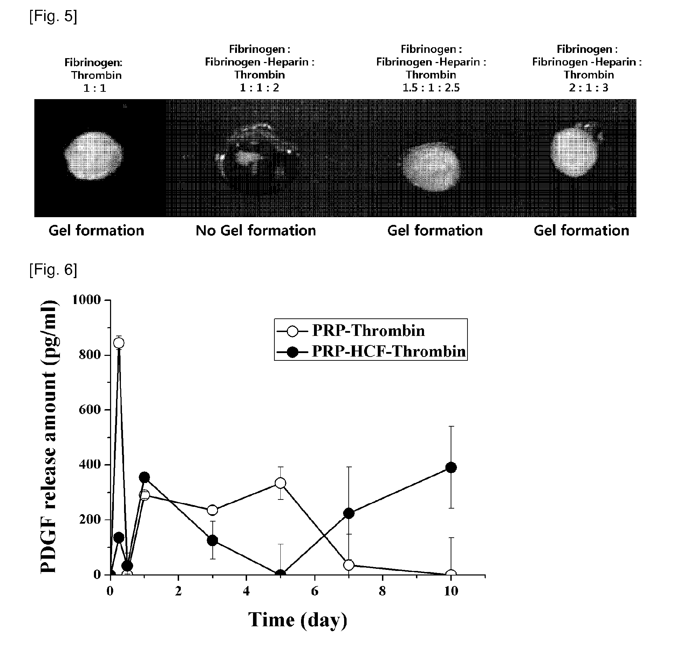

During the preparation of the fibrinogen mixture in the method of preparing the fibrinogen gel according to the present invention, a ratio of free fibrinogen to heparin-conjugated fibrinogen may be 1:1 to 5:1. Within this range, clot formation of a fibrin gel may be controlled. When the ratio is less than 1:1, a mechanical property of the gel is poor, and it is difficult to form a fibrin gel (refer to FIG. 5). If the gel is not formed, extended-release, which is an object of the present invention, may not be provided, and the gel having a very poor mechanical property may not provide long-term sustained release due to a large quantity of initial burst even when it is injected into a human body. On the other hand, when the ratio is greater than 5:1, the gel is well formed, but a relative amount of heparin mixed with fibrinogen in the fibrin gel decreases, and thus a quantity of growth factors conjugated therewith also decreases. As a result, the fibrin gel used to contain a therapeutically-effective dose of a protein drug increases in quantity, and efficiency in sustained-release of the protein drug drops.

In one embodiment, a kit for preparing a heparin-conjugated fibrin gel including heparin-conjugated fibrinogen, heparin-free fibrinogen and thrombin is provided. A ratio of the heparin-conjugated fibrinogen to the heparin-free fibrinogen in the kit may be 1:1 to 5:1. The heparin-conjugated fibrinogen, the heparin-free fibrinogen and the thrombin may be individually lyophilized and stored, and each of them may be dissolved in a buffer solution and then mixed together before preparing a gel according to the preparation method of the present invention. Preferably, components each dissolved in a buffer solution are easily mixed using a two-way syringe just before preparing a fibrin gel.

In another embodiment, a kit for preparing a heparin-conjugated fibrin gel is provided, including a growth factor conjugated with heparin in addition to heparin-conjugated fibrinogen, free fibrinogen not conjugated with heparin and thrombin. The growth factor may be selected from, but is not limited to, the group consisting of BMPs, vascular endothelial growth factors (VEGFs), transforming growth factors-β (TGFs-β), platelet-derived growth factors (PDGFs) and fibroblast growth factors (FGFs). In the present invention, at least one growth factor may be included, which may be, for example, a mixture created by mixing various kinds of growth factors such as a platelet-rich plasma (PRP).

How to control additional components or reaction conditions for preparing fibrin are well known to those skilled in the art. For example, to dissolve the fibrinogen mixture, an aprotinin solution may be used. Aprotinin serves to delay natural enzymatic decomposition of fibrin when the fibrin is prepared by crosslinking polymerization of fibrinogen and thrombin. In the present method, the thrombin may be dissolved in a solution containing calcium chloride. Depending on concentrations of the fibrinogen and thrombin solutions, a clot-formation time and clot firmness may be controlled. In this case, the quantity of dissolved thrombin may be the same as that of the fibrinogen mixture.

In another embodiment of the present invention, a growth factor carrier including a fibrin gel having heparin-directly conjugated fibrinogen, and at least one growth factor is provided. The fibrin gel prepared according to the present invention is bonded with the above-mentioned growth factor, and injected into a human body in the form of a fibrin gel composition, thereby releasing growth factors to a local site for a long period of time. Therefore, it can be used as a therapeutic drug stimulating regeneration of tissue such as bones, skin, blood vessels, cartilages, etc. Specifically, the fibrin gel composition prepared by the method of the present invention may be widely applied to treat bone defects, burns, diabetic or ischemic foot ulcers, ischemic heat diseases, limb ischemia, degenerative cartilage diseases, etc. through simple injection into a human body.

### Mode for the Invention

### Example 1: Activation of heparin

100 mg (0.00002 mole) of low-molecular weight heparin (molecular weight: 4000-6000; Sigma) was completely dissolved in 1 ml of 2-(N-morpholino)ethane sulfonic acid (MES, Sigma) buffer solution (0.05M, pH 6.0), and 0.0046 g of N-hydroxysuccinimide (NHS, 0.00008M) and 0.015336 g of N-(3-dimethylaminopropyl)-N-ethylcarbodiimide hydrochloride (EDC, 0.004M) were added thereto for 12-hour reaction at 4 °C, thereby resulting in preparing an NHS-heparin solution. The NHS-heparin solution was precipitated and extracted using acetone anhydride, and then lyophilized for 24 hours.

### Example 1-1: Conjugation of the activated heparin with fibrinogen,

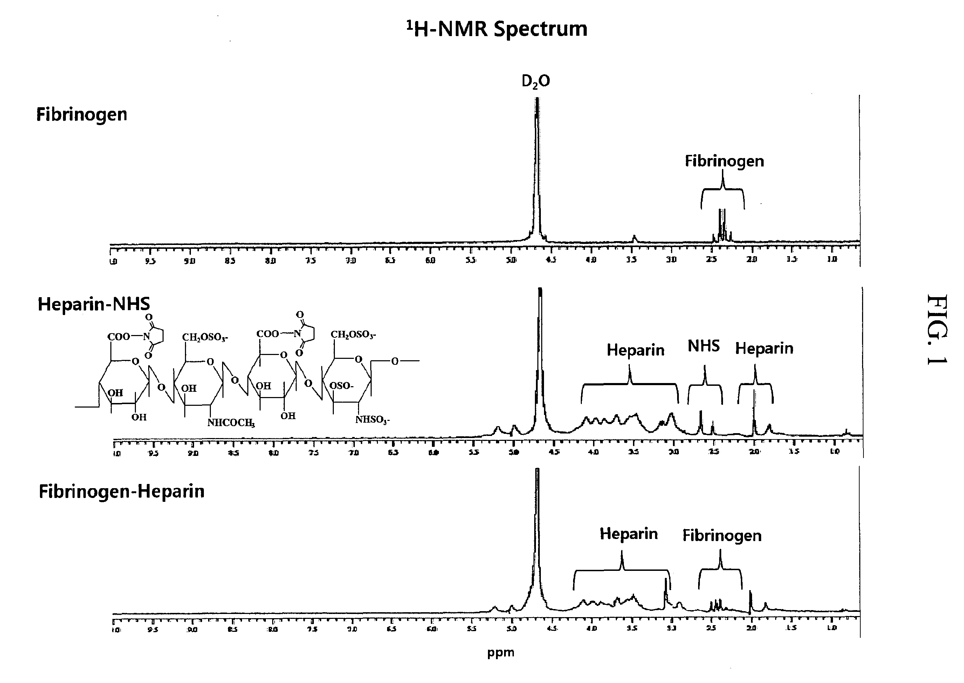

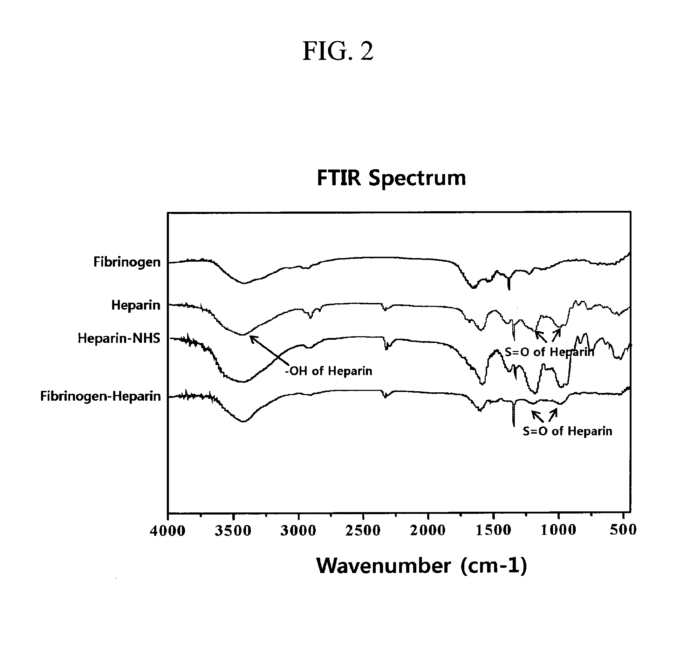

The lyophilized NHS-heparin (60 mg) prepared in Example 1 and human plasma-derived fibrinogen (100 mg) were completely dissolved in 20 ml of phosphate buffered saline (pH 7.4), and reacted for 3 hours at 4 °C. Here, the fibrinogen was slowly dissolved so as not to bubble. After the reaction, the resulting solution was precipitated using acetone anhydride and lyophilized in a dark room, thereby preparing heparin-conjugated fibrinogen. Conjugation of the activated heparin with fibrinogen was confirmed through NMR and FTIR analyses, and the analysis results are shown in FIGS. 1 and 2, respectively. Meanwhile, the heparin-conjugated fibrinogen prepared as described above was completely dissolved in 20 ml of phosphate buffered saline, and residual, unreacted heparin was eluted by dialyzing through a porous membrane bag (MWCO: 12-14000) for 24 hours at 4 °C while changing distilled water more than three times. The eluted heparin-conjugated fibrinogen was lyophilized for 48 hours in a dark room, thereby resulting in white powder of heparin-conjugated fibrinogen.

### Example 1-2: Quantification of heparin in heparin-conjugated fibrinogen

Heparin in heparin-conjugated fibrinogen prepared in Example 1-1 was quantified by toluidine blue analysis. Briefly, the heparin-conjugated fibrinogen was added to 2 ml of 0.02% NaCl aqueous solution contained in a test tube, and then 500µl of 0.005% toluidine blue-contained 0.001N HCl solution was added. The above mixture was incubated for 30 minutes, and gently shaken. 3 ml of n-hexane was added to the mixture and vigorously shaken for 10 to 15 seconds, followed by settling the mixture to stabilize layers. When removal of bubbles was confirmed, thereby stably separating an upper layer of hexane and a lower layer of water, about 200µl of sample was taken from the lower layer of water to subject to ELISA. Then, the sample was analyzed using a UV spectrophotometer at 630 nm. The content of heparin was calculated as 42.73 mg/g.

### Example 2: Preparation of fibrin gel containing bone morphogenetic protein

Bone morphogenetic protein (BMP) was dissolved in an aprotinin solution used as a protease inhibitor. The heparin-conjugated fibrinogen (33 mg) prepared in Example 1-1 was mixed with human plasma-derived fibrinogen (66 mg) commercially available at a ratio of 1:2, and dissolved in the above-mentioned aprotinin solution (1000 µl), thereby preparing a fibrinogen solution. Meanwhile, an equivalent amount of human plasma-derived thrombin (10 mg) was dissolved in a calcium chloride-dissolved solution (1000 µl), and mixed with the fibrinogen mixture solution, resulting in a BMP-conjugated fibrin gel (100 µl) protein carrier.

### Example 2-1: Release behavior of BMP in fibrin gel

Control group 1 was created by artificially mixing BMP with a heparin-free fibrin gel commercially available, and Control group 2 was created by mixing heparin and BMP with a fibrin gel commercially available. That is, in Control groups 1 and 2, the BMPs were conjugated with none or one of the fibrin gel and heparin.

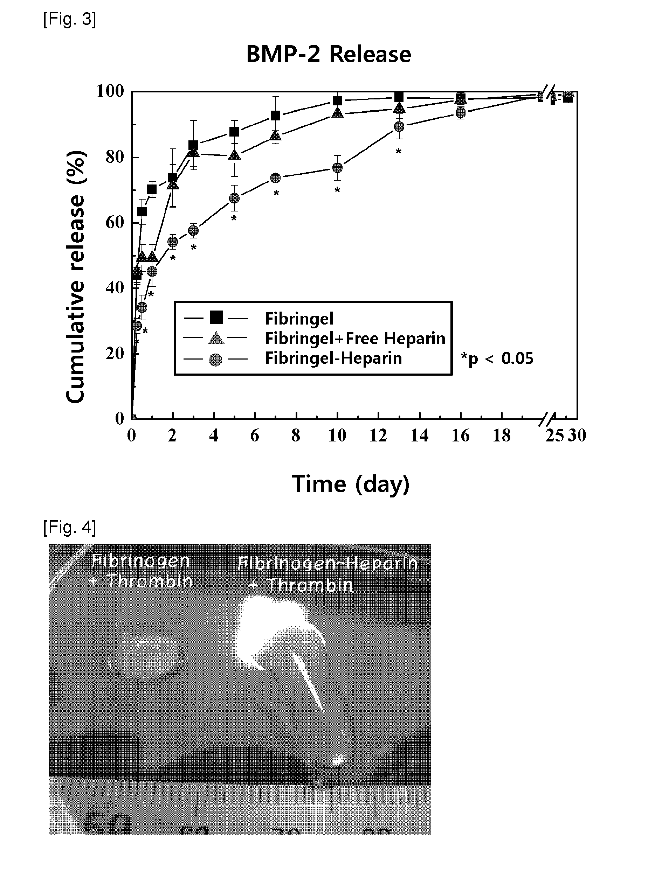

Meanwhile, the BMP-conjugated fibrin gel composition prepared in Example 2 as an experimental group was slowly stirred at 37 °C, and the BMP release behavior was examined for 28 days. The BMP release behavior is shown in the graph of FIG. 3. As shown in FIG. 3, it could be confirmed that the about 80% or more BMP was released from the fibrin gel compositions of control groups 1 and 2 in the first three days, whereas about 80% or more BMP was slowly released from the fibrin gel composition of the experimental group (Example 2 of the present invention), which was conjugated with heparin, for 13 days.

### Example 3: Evaluation of effect of free fibrinogen and heparin content on preparation of fibrin gel

Whether heparin-conjugated fibrinogen was individually capable of preparing a fibrin gel by the action of thrombin was examined.

Without mixing heparin-free fibrinogen, heparin-conjugated fibrinogen was prepared according to Example 1, and examined in degree of forming a gel using fibrin preparation conditions including thrombin. When a mixture of fibrinogen in an aprotinin buffer solution and a thrombin solution was polymerized using only heparin-free fibrinogen through crosslinking, gel formation was facilitated, but when the mixture was polymerized with only heparin-conjugated fibrinogen through crosslinking, a gel was not formed (Refer to FIG. 4). It could be confirmed in FIG. 4 that even when a formation plate was inclined after the gel forming reaction, the product using the heparin-free fibrinogen did not run, whereas the product using the heparin-conjugated fibrinogen could not make a gel, and thereby ran.

Afterwards, fibrin gels were prepared by mixing fibrinogens which were not conjugated with heparins before initiation of reactions (free fibrinogens) and heparin-conjugated fibrinogens at ratios of 1:1, 1.5:1 and 2:1, respectively. In FIG. 5, gels formed according to increasing contents of the heparin-free fibrinogen were shown. The formation of the gel was facilitated as the content of the heparin-free fibrinogen was increased.

### Example 4: Release behavior of platelet-derived growth factor in fibrin gel

1 ml of platelet-rich plasma (PRP) was mixed with 100 mg of fibrinogen mixture prepared by mixing the heparin-conjugated fibrinogen prepared in Example 1-1 and human plasma-derived fibrinogen commercially available at a ratio of 1:2, and human-plasma derived thrombin (10 mg) was dissolved in a calcium chloride-dissolved solution (1000 µl) and mixed with the fibrinogen mixture solution, resulting in 100 µl of PRP-conjugated fibrin gel protein carrier.

As a control group, a fibrin gel protein carrier prepared as described above except for using a heparin-free fibrin gel, which is commercially available, was used.

A PRP-thrombin fibrin gel and a PRP-heparin-conjugated fibrinogen (HCF) prepared in vitro, as experimental groups, were slowly stirred at 37 °C, and a release behavior of a platelet-derived growth factor (PDGF) was examined for 10 days. The release behavior of the PDGF is shown in the graph of FIG. 6. As shown in FIG. 6, it could be confirmed that the PDGF was mostly released from the control group fibrin gel during day 1 to day 6, whereas the PDGF was sustainably released from the experimental group, which was the PDGF-heparin-conjugated fibrin gel composition even on day 10 on a higher level than day 1.

According to a method of preparing a heparin-conjugated fibrin gel, a fibrin gel conjugated with heparin, which has an affinity for a drug such as a growth factor, can be easily prepared at low costs.

In addition, a heparin-conjugated fibrin gel prepared by the present method can be prepared as a fibrin gel composition containing a growth factor by binding the growth factor having an affinity for heparin. The fibrin gel composition prepared by the present method can effectively deliver a growth factor, and can thus be developed at low costs as a highly effective therapeutic drug capable of efficiently treating bone defects, burns, diabetic or ischemic foot ulcers, ischemic heat diseases, limb ischemia, and degenerative cartilage diseases through injection into a human body by sustainably releasing drugs such as growth factors, etc. to a local site for a long period of time.

While exemplary embodiments of the present invention have been shown and described, it will be appreciated by those skilled in the art that various changes can be made to the described exemplary embodiments without departing from the spirit and scope of the invention defined by the claims and their equivalents.

## Claims

1. A method of preparing a heparin-conjugated fibrin gel, comprising:
a) introducing an NHS group to heparin to form NHS-heparin;
b) directly conjugating the NHS-heparin with fibrinogen to prepare heparin-conjugated fibrinogen;
c) mixing free fibrinogen with the heparin-conjugated fibrinogen in step b) at a weight ratio of 1.5:1 to 5:1 to prepare a fibrinogen mixture; and
d) mixing thrombin with the fibrinogen mixture in step c).

2. The method according to claim 1, wherein the fibrin gel is an injectable gel.

3. The method according to claim 1, wherein the fibrinogen is mammal-derived fibrinogen.

4. The method according to claim 3, wherein the mammal is human.

5. The method according to claim 1, wherein the heparin has a molecular weight of about 1000 to about 20000.

6. The method according to claim 1, wherein the NHS-heparin is prepared by reaction between heparin, carbodiimide and N-hydroxysuccinimide in step a).

7. The method according to claim 1, wherein the heparin-conjugated fibrinogen in step b) is prepared by the reaction between a carboxyl group of NHS-heparin and an amino group present in protein of the fibrinogen.

8. A kit for preparing a heparin-conjugated fibrin gel, comprising:
heparin-conjugated fibrinogen;
free fibrinogen which is not conjugated with heparin; and
thrombin;
wherein the heparin-conjugated fibrinogen is fibrinogen directly conjugated with NHS-heparin, and the free fibrinogen and the heparin-conjugated fibrinogen are comprised at a weight ratio of 1.5:1 to 5:1.

9. The kit according to claim 8, further comprising a growth factor conjugated with heparin.

10. The kit according to claim 8, wherein the growth factor includes at least one selected from the group consisting of bone morphogenetic proteins (BMPs), vascular endothelial growth factors (VEGFs), transforming growth factors-β (TGFs-β), platelet-derived growth factors (PDGFs) and fibroblast growth factors (FGFs).

11. A growth factor carrier comprising a heparin-conjugated fibrin and at least one growth factor , wherein the heparin-conjugated fibrin gel is prepared from a mixture comprising heparin-conjugated fibrinogen, free fibrinogen and thrombin, wherein the heparin-conjugated fibrinogen is fibrinogen directly conjugated with NHS-heparin, and the free fibrinogen and the heparin-conjugated fibrinogen are comprised at a weight ratio of 1.5:1 to 5:1.

## Patentansprüche

1. Verfahren zur Herstellung eines Heparin-konjugierten Fibringels, umfassend:
a) das Einbringen einer NHS Gruppe in Heparin, um NHS-Heparin zu bilden;
b) die direkte Konjugation des NHS-Heparins mit Fibrinogen, um Heparin-konjugiertes Fibrinogen herzustellen;
c) das Mischen von freiem Fibrinogen mit dem Heparin-konjugierten Fibrinogen aus Schritt b) bei einem Gewichtsverhätniss von 1,5:1 bis 5:1, um ein Fibrinogengemisch herzustellen; und
d) das Mischen von Thrombin mit dem Fibrinogengemisch aus Schritt c).

2. Verfahren, gemäß Anspruch 1, wobei das Fibringel ein injizierbares Gel ist.

3. Verfahren, gemäß Anspruch 1, wobei das Fibrinogen ein von Säugetieren abgeleitetes Fibrinogen ist.

4. Verfahren, gemäß Anspruch 3, wobei das Säugetier ein Mensch ist.

5. Verfahren, gemäß Anspruch 1, wobei das Heparin ein Molekulargwicht von etwa 1000 bis etwa 20000 besitzt.

6. Verfahren, gemäß Anspruch 1, wobei das NHS-Heparin durch die Reaktion zwischen Heparin, Carbodiimid und N-Hydroxysuccinimid in Schritt a) hergestellt wird.

7. Verfahren, gemäß Anspruch 1, wobei das Heparin-konjugierte Fibrinogen in Schritt b) durch die Reaktion zwischen einer Carboxylgruppe des NHS-Heparins und einer Aminogruppe, die im Protein des Fibrinogens vorhanden ist, hergestellt wird.

8. Kit zur Herstellung eines Heparin-konjugierten Fibringels, umfassend:
Heparin-konjugiertes Fibrinogen;
freies Fibrinogen, welches nicht mit Heparin konjugiert ist; und
Thrombin;
wobei das Heparin-konjugierte Fibrinogen, Fibrinogen ist, das direkt mit NHS-Heparin konjugiert ist und das freie Fibrinogen und das Heparinkonjugierte Fibrinogen in einem Gewichtverhältniss von 1,5:1 bis 5:1 vorliegen.

9. Kit, gemäß Anspruch 8, das weiterhin einen Wachstumsfaktor umfasst, der mit Heparin konjugiert ist.

10. Kit, gemäß Anspruch 8, wobei der Wachstumsfaktor mindestens einen einschließt, ausgewählt aus der Gruppe aus Knochenmorphogenetischen Proteinen (BMPs), Vaskulären Endothelialen Wachstumsfaktoren (VEGFs), Transformierenden Wachstumsfaktoren-β (TGFs- ß), Blutplättchenwachstumsfaktoren (PDGFs) und Fibroblasten-Wachstumsfaktoren (FGFs).

11. Wachstumsfaktorenträger, umfassend ein Heparin-konjugiertes Fibrin und mindestens einen Wachstumsfaktor, wobei das Heparin-konjugierte Fibringel aus einem Gemisch umfassend Heparin-konjugiertes Fibrinogen, freies Fibrinogen und Thrombin hergestellt ist, wobei das Heparin-konjugierte Fibrinogen, Fibrinogen ist, das direkt mit NHS-Heparin konjugiert ist und das freie Fibrinogen und das Heparinkonjugierte Fibrinogen in einem Gewichtverhältniss von 1,5:1 bis 5:1 vorliegen.

## Revendications

1. Procédé de préparation d'un gel de fibrine conjugué à de l'héparine, comprenant :
a) l'introduction d'un groupe NHS sur de l'héparine afin de former un composé NHS-héparine ;
b) la conjugaison directe du composé NHS-héparine avec du fibrinogène afin de préparer du fibrinogène conjugué à de l'héparine ;
c) le mélange de fibrinogène libre avec le fibrinogène conjugué à de l'héparine de l'étape b) suivant un rapport en poids de 1,5:1 à 5:1 afin de préparer un mélange de fibrinogène ; et
d) le mélange de thrombine avec le mélange de fibrinogène de l'étape c).

2. Procédé selon la revendication 1, dans lequel le gel de fibrine est un gel pouvant être injecté.

3. Procédé selon la revendication 1, dans lequel le fibrinogène est du fibrinogène dérivé de mammifère.

4. Procédé selon la revendication 3, dans lequel le mammifère est humain.

5. Procédé selon la revendication 1, dans lequel l'héparine présente un poids moléculaire de 1.000 environ à 20.000 environ.

6. Procédé selon la revendication 1, dans lequel le composé NHS-héparine est préparé par réaction entre de l'héparine, du carbodiimide et du N-hydroxysuccinimide (NHS) à l'étape a).

7. Procédé selon la revendication 1, dans lequel le fibrinogène conjugué à de l'héparine de l'étape b) est préparé par la réaction entre le groupe carboxyle du composé NHS-héparine et un groupe amino présent dans la protéine du fibrinogène.

8. Kit de préparation d'un gel de fibrine conjugué à de l'héparine, comprenant :
du fibrinogène conjugué à de l'héparine ;
du fibrinogène libre qui n'est pas conjugué à de l'héparine ; et
de la thrombine ;
dans lequel le fibrinogène conjugué à de l'héparine est du fibrinogène conjugué directement au composé NHS-héparine, et le fibrinogène libre et le fibrinogène conjugué à de l'héparine sont contenus suivant un rapport en poids de 1,5:1 à 5:1.

9. Kit selon la revendication 8, comprenant en outre un facteur de croissance conjugué à de l'héparine.

10. Kit selon la revendication 8, dans lequel le facteur de croissance comporte au moins un élément sélectionné à partir du groupe constitué par des protéines morphogénétiques d'os (BMP), des facteurs de croissance de l'endothélium vasculaire (VEGF), des facteurs de croissance de transformation b (TGF-b), des facteurs de croissance dérivés de plaquettes (PDGF) et des facteurs de croissance des fibroblastes (FGF).

11. Support de facteur de croissance comprenant de la fibrine conjuguée à de l'héparine et au moins un facteur de croissance, dans lequel le gel de fibrine conjugué à de l'héparine est préparé à partir d'un mélange comprenant du fibrinogène conjugué à de l'héparine, du fibrinogène libre et de la thrombine, dans lequel le fibrinogène conjugué à de l'héparine est du fibrinogène conjugué directement avec du composé NHS-héparine, et le fibrinogène libre et le fibrinogène conjugué à de l'héparine sont contenus suivant un rapport en poids de 1,5:1 à 5:1.
